(19) Europäisches Patentamt
European Patent Office
Office européen des brevets

(11) Veröffentlichungsnummer: **0 229 622 B1**

(12) **EUROPÄISCHE PATENTSCHRIFT**

(45) Veröffentlichungstag der Patentschrift: **18.07.90**

(51) Int. Cl.[5]: **C 07 D 317/36**, C 08 G 71/00

(21) Anmeldenummer: **87100074.1**

(22) Anmeldetag: **07.01.87**

# (54) Verfahren zur Herstellung von 2-Oxo-1,3-dioxolanen.

(30) Priorität: **11.01.86 DE 3600602**

(43) Veröffentlichungstag der Anmeldung:
**22.07.87 Patentblatt 87/30**

(45) Bekanntmachung des Hinweises auf die Patenterteilung:
**18.07.90 Patentblatt 90/29**

(84) Benannte Vertragsstaaten:
**AT BE CH DE ES FR GB IT LI NL SE**

(56) Entgegenhaltungen:
**WO-A-84/03701**
**DE-A-2 611 087**
**US-A-4 663 467**

(73) Patentinhaber: **HOECHST AKTIENGESELLSCHAFT**
**Postfach 80 03 20**
**D-6230 Frankfurt am Main 80 (DE)**

(72) Erfinder: **Brindöpke, Gerhard, Dr.**
**Loreleistrasse 18**
**D-6230 Frankfurt am Main 80 (DE)**

**EP 0 229 622 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Es ist bekannt, daß man durch Umsetzung von Alkylenoxiden mit Kohlendioxid in Gegenwart von Katalysatoren 2-Oxo-1,3-dioxolane (auch Alkylencarbonate genannt) erhalten kann. In der DE—OS 26 11 087 wird ein Verfahren zur Herstellung von Alkylencarbonaten der allgemeinen Formel

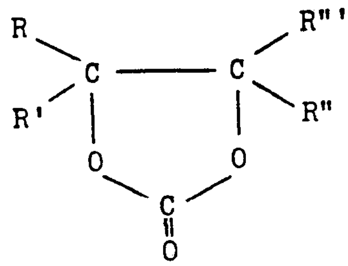

(1)

beschrieben, wobei ein Alkylenoxid mit $CO_2$ in Gegenwart eines Katalysators, der aus einer Kombination einer protischen Substanz der Formel ROH und einer stickstoffhaltigen Base besteht, bei Temperaturen zwischen 0 und 200°C und einem Druck von 1 bis 98 bar umgesetzt wird. Protische Substanzen sind Wasser, Alkohole und Phenol. Als stickstoffhaltige Basen werden Trimethylamin, Triethylamin, Pyridin oder Dimethylanilin angeführt. Bezüglich der Substituenten R bis R''' wird nur allgemein angegeben, daß diese Wasserstoff oder ein Alkyl-, Aryl, Cycloalkyl- oder Aralkylrest sein können. Nähere Angaben werden nicht gemacht. In den Beispielen werden nur Ethylenoxid und Propylenoxid als Alkylenoxide angeführt und es wird stets unter Druck gearbeitet (vorzugsweise 10 bar).

G. Rokicki und Mitarbeiter beschreiben in "Monatshefte für Chemie" *115* (1984), 205—214, die Herstellung von cyclischen Carbonaten aus $CO_2$ und Oxiranen in Gegenwart von Alkalimetallsalz-Phasentransfer-Katalysatoren. Als Phasentransfermittel werden Kronenether, 1,4-Diazabicyclo-[2,2,2]-octan (DABCO), N,N,N,N-Tetramethylethylendiamin (TMEDA) und Triethylbenzylammoniumchlorid (TEBA), aber auch — mit verringerter Ausbeute — Polyethylenglykol eingesetzt. Hohe Ausbeuten, d.h. fast theoretische Werte, werden nur durch Anfangsdrücke von 40 bar erreicht. Beim Arbeiten bei 6 bar wird eine um ca. 25% niedrigere Ausbeute, bei 1 bar sogar nur 8% Ausbeute erhalten. Eingesetzte Alkalimetallsalze sind Alkalihalogenide und -carbonate. Als Epoxidkomponenten werden Ethylen- und Propylenoxid, Epihalogenhydrine. Glycid, N-Butyl, Allyl, und Phenylglycidether, Styroloxid und 3,3-disubstituiertes Cyclohexenoxid angeführt.

Eine weitere Arbeit von G. Rokicki (Makromol. Chem. *186,* 331—337 (1985)) beschreibt die Herstellung von cyclischen Dicarbonaten durch Einsatz von 2,2-Bis[4-(2,3-epoxypropoxy)phenyl]propan bzw. einem Epoxidharz (® Epikote 828) unter den vorstehend angegebenen Bedingungen.

Es ist weiterhin bekannt (PCT WO 84/03,701), Alkylencarbonate durch Behandlung von Alkylenoxiden mit $CO_2$ in Gegenwart eines Alkohols wie Methanol und einer (un-)substituierten Phosphinverbindung als Katalysator herzustellen. Auch in diesem Falle wird bei erhöhtem Druck (21 bar) gearbeitet. Darüberhinaus ergibt sich aus der Veröffentlichung, daß die Gegenwart sowohl des Alkohols als auch des Phosphins zwingend ist, um eine gute Ausbeute zu erhalten.

Aus dem Stand der Technik ergibt sich also, daß zur Erzielung einer hohen Ausbeute stets bei hohen Drucken gearbeitet werden muß und/oder daß die Gegenwart einer protischen Substanz zwingend ist, um zufriedenstellende Ausbeuten zu erzielen.

Die genannten Nachteile können gemäß der vorliegenden Erfindung umgangen werden.

Gegenstand der Erfindung ist daher ein Verfahren zur Herstellung von 2-Oxo-1,3-dioxolanen durch Umsetzung von Epoxiden mit Kohlendioxid in Gegenwart von Alkalijodiden, das dadurch gekennzeichnet ist, daß mindestens eine Epoxidverbindung in Anwesenheit oder Abwesenheit eines inerten Lösungsmittels entweder mit einer Kombination aus A) mindestens einem Alkalijodid und B) mindestens einer Verbindung der Gruppe a) Polyol und b) einem Ether oder Polyether mit den Strukturen

$$HO-(-\underset{R_1}{CH}-CH_2-O)_n-(-\underset{R}{CH}-CH_2-O-)_m-H \qquad n > 1,\ m \geqslant 0 \qquad B_a)$$

und

$$R-O-(-\underset{R_1}{CH}-CH_2-O-)_n-(-\underset{R_2}{CH}-CH_2-O-)_m-R_3 \qquad n > 1,\ m \geqslant 0 \qquad B_b)$$

in denen

R gleich oder verschieden und Alkyl, Aralkyl mit jeweils 1—10 C-Atomen in der Alkylgruppe oder Aryl ist,

$R_1$, $R_2$ gleich oder verschieden und Wasserstoff oder Alkyl mit 1—10 C-Atomen sind und

$R_3$ gleich R ist, jedoch auch Wasserstoff sein kann, oder, wenn die Epoxidverbindung die Strukturmerkmale der Verbindungen Ba) oder Bb) bereits aufweist, mit dem Alkalijodid A) allein, bei Temperaturen von 40 bis 180°C unter Einleitung von Kohlendioxid bei Normaldruck oder geringfügig erhöhtem Druck zu den entsprechenden organischen Carbonaten umgesetzt wird. Nach diesem Verfahren können die Epoxygruppen in den Ausgangsverbindungen teilweise oder vollständig zur Reaktion gebracht werden.

Der Vorteil des Verfahrens ist die Anwendung von Normal-oder schwach erhöhtem Druck, wobei kein großer apparativer Aufwand erforderlich ist. Weiterhin ist die hohe Selektivität der Umsetzung hervorzuheben, d. h. es treten praktisch keine Epoxid-Nebenreaktionen wie Homopolymerisation ein, die bei diesem Reaktionsmechanismus im Stand der Technik beschrieben worden sind. Ferner ist es durch das Verfahren möglich, lagerstabile Epoxid-/Carbonatgemische herzustellen, die eine Multifunktionalität aufweisen und für viele Anwendungsgebiete verfügbar sind. Die Neutralität des verwendeten Katalysators führt zudem zu keiner Inhibierung von möglichen Folgereaktionen an der Epoxidgruppe bzw. an der Carbonatgruppe z.B. bei einer durch Säure katalysierten Veretherungs- oder Veresterungsreaktion der Epoxidgruppe.

Der bei dem Verfahren anzuwendende Druck beträgt im allgemeinen 1 bis 10, vorzugsweise 1 bis 5 und insbesondere 1 bis 3 bar. In den meisten Fällen wird Normaldruck angewandt, gegebenenfalls kann aber auch unter erhöhtem Druck gearbeitet werden. Der bevorzugte Temperaturbereich des Verfahrens liegt bei 60 bis 180, insbesondere 80 bis 150°C.

Als Epoxidkomponenten, die mit $CO_2$ umgesetzt werden können und im allgemeinen mindestens eine endständige Epoxygruppe besitzen, eignen sich beispielsweise folgende Verbindungen:

Aliphatische Epoxide mit mindestens 6 C-Atomen wie Hexen-, Octen-, Dodecen-1-oxid, Glycidol und Epihalogenhydrine der Formel

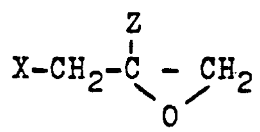

(2),

in der Z ein Wasserstoffatom, eine Methyl- oder Ethylgruppe und X ein Halogenatom oder eine OH-Gruppe darstellt. Beispiele für derartige Epihalogenhydrine sind Epichlorhydrin, Epibromhydrin, 1,2-Epoxi-2-methyl-3-chlorpropan und 1,2-Epoxi-2-ethyl-3-chlorpropan.

Weitere Epoxykomponenten, die erfindungsgemäß eingesetzt werden können, schließen z.B. Epoxykomponenten, die im Durchschnitt mindestens eine endständige 1,2-Epoxygruppe aufweisen ein. Vorzugsweise sind dies Epoxyverbindungen, die im Durchschnitt mindestens eine substituierte oder nicht substituierte Glycidylethergruppe oder eine substituierte oder nicht substituierte Glycidylestergruppe enthalten, ferner epoxydierte, mehrfach ungesättigte Verbindungen und amid- oder urethangruppenhaltige Epoxide.

Epoxyverbindungen, die im Durchschnitt mindestens eine substituierte oder nicht substituierte Glycidylethergruppe enthalten, welche die Formel

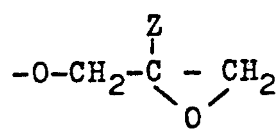

(3),

aufweist, in der Z Wasserstoff, eine Methyl- oder eine Ethylgruppe darstellt, sind z. B. Glycidyl- oder Polyglycidylether von ein- oder mehrwertigen Alkoholen, Phenol oder mehrwertigen Phenolen, die ein oder mehrere aromatische Kerne aufweisen sowie von Novolaken, Polyglycidylether von alkoholischen Polyhydroxylverbindungen, erhalten durch Additionsreaktion von mehrwertigen Phenolen, enthaltend einen oder mehrere aromatische Kerne mit Alkylenoxiden, die 2 bis 4 C-Atome aufweisen, und Polyglycidylether von alkoholischen Polyhydroxylverbindungen, die einen oder mehrere alicyclische Ringe aufweisen. Als Phenole werden beispielsweise eingesetzt Phenol, die verschiedenen Kresole, Resorcin, Hydrochinon, Pyrogallol, Phloroglycin, 1,5-, 2,7-, 2,6-Dihydroxynaphthaline und ähnliche, 2,2-Bis-(p-hydroxy-phenyl)-propan und -methan (bekannt als Bisphenol A bzw. F), 2,4'-Dihydroxydiphenylmethan u.ä.. Mehrwertige Alkohole, die zu Glycidylethern umgesetzt werden können, sind beispielsweise Ethylenglykol, Propylenglykol, Butylenglykol, Neopentylglykol, Hexylenglykol, Polyethylenglykol, Polypropylenglykol u.ä.. Entsprechende einwertige Alkohole sind Ethanol, n-Butanol und Ethylhexanol.

Hierzu werden auch plastifizierte Epoxidharze mit endständigen Epoxygruppen gerechnet, die durch Teilumsetzung der Epoxygruppen von mindestens zwei Epoxygruppen enthaltenden Epoxidharzen mit OH- und COOH-haltigen Substanzen wie mehrwertigen Alkoholen z.B. den obengenannten Diolen, Polycarbonsäuren oder Carboxyl- oder OH-gruppenhaltigen Polyestern hergestellt werden.

Weitere Epoxyverbindungen sind Glycidylester von gesättigten oder ethylenisch ungesättigten

Carbonsäuren mit mindestens einer substituierten oder nicht substituierten Glycidylestergruppe der folgenden Formel

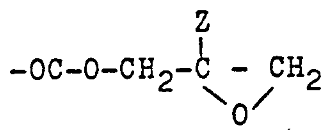

(4),

in der Z ein Wasserstoffatom, eine Methyl- oder eine Ethylgruppe darstellt. Die Säuren sind aliphatische oder aromatische, gasättigte oder ungesättigte Mono- oder Polycarbonsäuren, z. B. Acrylsäure, Methacrylsäure, Adipinsäure, die verschiedenen Phthalsäuren, Tetra- und Hexahydrophtalsäure u.ä. Ein sehr gebräuchlicher Glycidylester ist im Handel erhältlich und stellt den Glycidylester einer Mischung von gesättigten Monocarbonsäuren mit einer Kettenlänge von 9 bis 11 Kohlenstoffatomen dar, bestehend hauptsächlich (ungefähr 94%) aus tertiären Säuren (Versaticsäureglycidylester). Hier eingeschlossen sind auch Epoxidharze, die durch Copolymerisation von Glycidylmethacrylsäureester mit anderen copolymerisierbaren Monomeren wie Styrol und (Meth-)Acrylsäureestern erhalten worden sind.

Des weiteren sind amid- oder urethangruppenhaltige Epoxide für die Umsetzung geeignet, z. B. Triglycidylisocyanurat oder glycidolverkapptes Hexamethylendiisocyanat. Mischungen der genannten Epoxidverbindungen können ebenfalls eingesetzt werden.

In der Kombination, die die Umsetzung der Epoxide mit $CO_2$ katalytisch beeinflußt, ist die Komponente A) mindestens ein Alkalijodid wie Kalium-, Natrium- und Lithiumjodid. Verbindungen B) besitzen die allgemeinen Strukturen

$$HO-(-\underset{R_1}{CH}-CH_2-O)_n-(-\underset{R}{CH}-CH_2-O-)_m-H \qquad n > 1,\ m \geqslant 0 \qquad Ba)$$

und

$$R-O-(-\underset{R_1}{CH}-CH_2-O-)_n-(-\underset{R_2}{CH}-CH_2-O-)_m-R_3 \qquad n \geqslant 1,\ m \geqslant 0 \qquad Bb)$$

in denen

R gleich oder verschieden und Alkyl, Aralkyl mit jeweils 1—10 C-Atomen in der Alkylgruppe oder Aryl ist,

$R_1$, $R_2$ gleich oder verschieden und Wasserstoff oder Alkyl mit 1—10 C-Atomen sind und

$R_3$ gleich R ist, jedoch auch Wasserstoff sein kann.

In $B_a$) bedeuten n vorzugsweise 2 bis 40 und insbesondere 2 bis 20, und m vorzugsweise 0 bis 40, insbesondere 0 bis 20. Die Summe aus n und m steht vorzugsweise für 2 bis 40.

In $B_b$) bedeuten n vorzugsweise 1 bis 40, insbesondere 1 bis 20, und m vorzugsweise 0 bis 40, insbesondere 0 bis 20. Die Summe aus n und m steht hier vorzugsweise für 1 bis 40.

Beispiele für Verbindungen Ba) sind z. B. Polyethylenglykole und Polypropylenglykole, wie Diethylen-, Triethylen-, Tetraethylen- und Dipropylenglykol.

Beispiele für Verbindungen Bb) sind z. B. Ether oder Polyether wie Ethylenglykolmonomethyl- und -monobutylether, Dimethoxyethan, Diethylenglykolmonomethyl- und -monobutylether, Diethylenglykoldimethyl- und diethylether, Triethylenglykolmono- und -dimethylether, Propylenglykolmonobutylether sowie ein Monobutylether eines Polyglykols, zu dessen Herstellung ein Gemisch aus Ethylen- und Propylenoxid verwendet wurde.

Die Verbindungen Ba) und Bb) werden jeweils in Mengen von 1—95, vorzugsweise 1—50, insbesondere 10—30 Gew.%, bezogen auf die Epoxidkomponente eingesetzt.

Wenn die umzusetzenden Epoxide die bei den Verbindungen Ba) und Bb) genannten Strukturmerkmale selbst aufweisen, kann auf den Einsatz einer Verbindung des Typs Ba) oder Bb) verzichtet werden.

Beispiele für derartige Epoxide sind Glycidylether von Di-, Tri- oder Polyethylen- oder propylglykolen, Diglycidylether des Diethylenglykols, Polypropylen- oder Polyethylenglykols. Andererseits können bei diesem Verfahren diese Epoxide, da sie die benötigten Strukturmerkmale aufweisen, die Aufgaben der Verbindungen Ba) und Bb) bei der Carbonatisierung von Epoxiden übernehmen, die keine Polyethergruppen enthalten. In diesem Falle können z.B. Gemische mit den vorstehend genannten Epoxidverbindungen bei dem erfindungsgemäßen Verfahren vorliegen.

Die Alkalijodide A) werden im allgemeinen einzeln oder im Gemisch in Mengen von 0,01 bis 1, vorzugsweise 0,05 bis 1 und insbesondere 0,05 bis 0,5 Gewichtsprozent, bezogen auf das Gewicht der Epoxidkomponente, eingesetzt.

Die Reaktionszeit kann in weiten Grenzen schwanken. Im allgemeinen wird die Umsetzung so geführt, daß die Epoxidgruppen praktisch vollkommen umgesetzt sind. Die Reaktion wird beispielsweise durch

Titration der Epoxidgruppen verfolgt und an dem Punkt abgebrochen, der im Rahmen der Analysengenauigkeit als "epoxidgruppenarm bzw. -frei" angesehen wird. Man erhält auf diese Weise Alkylcarbonate aus beliebigen Epoxidverbindungen, die in bekannter Weise weiterverarbeitet werden können.

Darüberhinaus kann man die Reaktion bei Vorliegen von Polyepoxiden an jedem gewünschten Punkt abbrechen, so daß Verbindungen erhalten werden, die neben Carbonatgruppen noch intakte Epoxidgruppen aufweisen. Letzteres hat den Vorteil, daß je nach Art der gewünschten Weiterverarbeitung und des Einsatztes der Produkte eine selektive Umsetzung der Epoxid- neben der Carbonatgruppe — und umgekehrt — erfolgen kann. Im allgemeinen wird man bei den Polyepoxiden (Anzahl der Epoxidgruppen $n \geq 2$) die Umsetzung in diesem Fall so durchführen, daß der Anteil der umgesetzten Epoxidgruppen 0,1—0,9 n beträgt.

Die Umsetzung der Epoxyidverbindungen mit $CO_2$ kann in Anwesenheit oder Abwesenheit von Lösungsmitteln erfolgen. Im allgemeinen werden keine Lösungsmittel eingesetzt, wenn die Epoxidverbindungen oberhalb 50°C im flüssigen Zustand vorliegen. Stellen sie jedoch bei der Reaktionstemperatur zähflüssige Schmelzen dar und erschweren dadurch eine homogene Verteilung des Kohlendioxids beim Rühren oder ist eine Weiterverarbeitung des Reaktionsproduktes in Lösung vorgesehen, werden im allgemeinen Lösungsmittel verwendet.

Als Lösungsmittel können aromatisches Kohlenwasserstoffe wie Toluol, Xylol und bei der Erdölcrackung anfallende Kohlenswasserstoffgemische, Dioxan, Tetrahydrofuran, und andere gegen Epoxigruppen inerte Lösungsmittel eingesetzt werden.

Die nach dem erfindungsgemäßen Verfahren erhaltenen Produkte werden zur Herstellung von Urethangruppen enthaltenden Kunstharzen in Form von Überzügen oder Formkörpern verwendet.

In den nachfolgenden Beispielen bedeutet T stets Gewichtsteile und % stets Gewichts-%.

## Beispiele

Allgemeine Vorschrift zur Herstellung von Carbonaten aus Epoxiden

In einer mit Rührer, Thermometer und einem Gaseinleitungsrohr bestückten Apparatur (gegebenenfalls Druckapparatur) wurde das Epoxid mit dem in der Tabelle angeführten Epoxidgehalt vorgelegt und nach Zugabe des Katalysators A) und der Komponente B) unter kräftigem Rühren und unter Einleiten von Kohlendioxid auf die angegebene Reaktionstemperatur erhitzt. Gegebenenfalls wurde die Reaktionsapparatur vorher mit Kohlendioxid ausgespült.

Unter fortlaufendem Einleiten von Kohlendioxid wurde unter Normaldruck von 1 bar bei den genannten Reaktionstemperaturen bis zum gewünschten Restepoxidgehalt, der durch Titration ermittelt wird, nachgerührt und evtl. das Lösungsmittel abdestilliert Anschließend wurde bei Vorhandensein einer Trübung in der Hitze filtriert. Die Ausbeute bezieht sich auf den Umsatzt, der durch den Restepoxidgehalt bestimmt wird.

In der Tabelle bedeuten:

®Beckopox EP 140 technischer Diglycidylether des Bisphenol A (Handelsbezeichnung der Fa. HOECHST AG)

®Epicote 1001 technischer Diglycidylether des Bisphenol A (Handelsbezeichnung der Fa. DOW, USA)

®Denacol EX 861 Polyethylenglykoldiglycidylether

EX830

EX 920 Polypropylenglykoldiglycidylether (Handelsbezeichnungen der Firma Nagase, Japan)

®Cardura E 10 Glycidylester der Versaticsäure (Handelprodukt der Firma Shell)

Beckopox 080 Glycidylether des Ethylhexanols (Handelsbezeichnung der Fa. HOECHST AG)

Butylglykol Ethylenglykolmonobutylether

DMDG Diglykoldimethylether

DMTG Triglykoldimethylether

TEG Triethylenglykol

B11/50 Polyglykol B11/50 (Basis Ethylen-Propylenoxid) (Bez. der Fa. HOECHST AG,)

DEGMM Diethylenglykolmonomethylether

PE 300 Polyethylenglykol PE 300 (mittl. Molmasse 300)

| Bsp. | Epoxid | Epoxid gen. (%) | Menge (T) | Katalysator | Menge (T) | % | Etherglykol | Menge (T) | Temp. (°C) | Reaktionszeit (h) | Ausbeute % | Epoxidgeh. (%) | geb. $CO_2$ (%) |
|---|---|---|---|---|---|---|---|---|---|---|---|---|---|
| 1 | Beckopox EP 140 | 8,6 | 511 | KJ | 0,77 | 0,15 | DMTG | 270 | 140 | 11 | 98,1 | 0,19 | 18,5 |
| 2 | " | 8,6 | 1002 | KJ | 1 | 0,1 | DMDG | 140 | 140 | 12 | 92,4 | 3,09 | 12,0 |
| 3 | " | 8,6 | 513 | NaJ | 0,77 | 0,15 | DMTG | 271 | 140 | 14 | 92,8 | 0,18 | 18,3 |
| 4 | " | 8,6 | 565 | KJ | 0,85 | 0,15 | 1-Methoxy-propanol | 170 | Rückfluß | 15 | 96,1 | 0,24 | 18,1 |
| 5 | " | 8,6 | 970 | KJ | 1,48 | 0,15 | DMDG | 512 | 140 | 17 | 93,7 | 0,25 | 18,1 |
| 6 | " | 8,6 | 924 | KJ | 1,39 | 0,15 | Butyl-glykol | 488 | 140 | 13 | 92,3 | 0,15 | 18,0 |
| 7 | " | 8,6 | 972 | KJ | 1,49 | 0,15 | TEG | 513 | 140 | 11 | 91,6 | 0,18 | 18,2 |
| 8 | " | 8,6 | 919 | KJ | 1,40 | 0,15 | B11/50 | 485 | 140 | 9 | 90,2 | 0,2 | 18,0 |
| 9 | " | 8,6 | 928 | KJ | 1,42 | 0,15 | DEGMM | 490 | 140 | 10 | 93,2 | 0,1 | 18,5 |
| 10 | " | 8,6 | 517 | KJ | 0,78 | 0,15 | PE 300 | 273 | 140 | 10 | 97,9 | 0,2 | 18,4 |
| 11 | Epicote 1001 | 3,33 | 817 | KJ | 0,8 | 0,1 | DMTG | 381 | 140 | 10 | 94,2 | 0,1 | 8,0 |
| 12 | " " | 3,33 | 1000 | KJ | 1 | 0,1 | DMDG | 262 | 120 | 6 | 95,4 | 1,63 | 4,0 |
| 13 | " " | 3,33 | 1000 | KJ | 1 | 0,1 | DMDG | 468 | 140 | 13 | 96,2 | 0,1 | 7,9 |
| 14 | Denacol EX 861 | 2,42 | 980 | KJ | 1 | 0,1 | DMDG | 420 | 130 | 12 | 95,2 | 0,05 | 6,0 |
| 15 | " " 920 | 9,25 | 989 | KJ | 1 | 0,1 | – | – | 120 | 15 | 92,6 | 0,1 | 19,4 |
| 16 | " " 920 | 9,25 | 1509 | KJ | 1,51 | 0,1 | – | – | 120 | 5 | 95,2 | 4,15 | 10,7 |
| 17 | " " 830 | 6,20 | 992 | KJ | 1 | 0,1 | – | – | 140 | 13 | 94,1 | 0,1 | 14,0 |
| 18 | " " 830 | 6,2 | 1030 | KJ | 1,03 | 0,1 | – | – | 120 | 4 | 96,2 | 2,88 | 7,6 |
| 19 | Cardura E 10 | 6,9 | 500 | KJ | 0,75 | 0,15 | DEGMM | 214 | 140 | 18 | 95,2 | 0,15 | 14,3 |

Tabelle (Fortsetzung)

| Bsp. | Epoxid | Epoxid geh. (%) | Menge (T) | Kata-lysator | Menge (T) | % | Ether-glykol | Menge (T) | Temp. (°C) | Reaktions-zeit (h) | Ausbeute % | Epoxidgeh. (%) | geb. $CO_2$ (%) |
|---|---|---|---|---|---|---|---|---|---|---|---|---|---|
| 20 | Dodecen-1-oxid | 8,7 | 409 | KJ | 0,61 | 0,15 | TEG | 175 | 140 | 14 | 91,6 | 0,21 | 18,4 |
| 21 | Beckopox 080 | 8,5 | 507 | KJ | 0,75 | 0,15 | DEGMM | 214 | 140 | 14 | 95,8 | 0,13 | 18,3 |
| 22 | n-Butylglycidyl-ether<br>Denacol EX 830 | 12,3<br>6,2 | 432<br>108 | KJ | 2,7 | 0,5 | - | - | 130 | 15 | 95,9 | 0,25 | 22,1 |
| 23 | Beckopox EP 140<br>Denacol EX 830 | 8,6<br>6,2 | 532<br>228 | KJ | 3,8 | 0,5 | - | - | 140 | 14 | 95,8 | 0,15 | 17,0 |
| 24 | Glydidyl-methacrylat | 11,3 | 840 | KJ | 4,2 | 0,5 | 1-Methoxy-propanol | 360 | 80 | 21 | 94,5 | 0,4 | 22,4 |

**Patentansprüche**

1. Verfahren zur Herstellung von 2-Oxo-1,3-dioxolanen durch Umsetzung von Epoxiden mit Kohlendioxid in Gegenwart von Alkalijodiden, dadurch gekennzeichnet, daß mindestens eine Epoxidverbindung in Anwesenheit oder Abwesenheit eines inerten Lösungsmittels entweder mit einer Kombination aus A) mindestens einem Alkalijodid und B) mindestens einer Verbindung der Gruppe a) Polyol und b) einem Ether oder Polyether mit den Strukturen

$$HO-(-\underset{R_1}{CH}-CH_2-O)_n-(-\underset{R}{CH}-CH_2-O-)_m-H \qquad n>1,\ m\geqslant 0$$

und

$$R-O-(-\underset{R_1}{CH}-CH_2-O-)_n-(-\underset{R_2}{CH}-CH_2-O-)_m-R_3 \qquad n\geqslant 1,\ m\geqslant 0$$

Ba)

Bb)

in denen

R gleich oder verschieden und Alkyl, Aralkyl mit jeweils 1—10 C-Atomen in der Alkylgruppe oder Aryl ist,

$R_1$, $R_2$ gleich oder verschieden und Wasserstoff oder Alkyl mit 1—10 C-Atomen sind und

$R_3$ gleich R ist, jedoch auch Wasserstoff sein kann, oder, wenn die Epoxidverbindung die Strukturmerkmale der Verbindungen Ba) oder Bb) bereits aufweist, mit dem Alkalijodid A) allein, bei Temperaturen von 40 bis 180°C unter Einleitung von Kohlendioxid bei Normaldruck oder geringfügig erhöhtem Druck, zu den entsprechenden organischen Carbonaten umgesetzt wird.

2. Verfahren nach Anspruch 1, dadurch gekennzeichnet, daß bei einem Druck von 1 bis 10, vorzugsweise 1 bis 5, insbesondere 1 bis 3 bar gearbeitet wird.

3. Verfahren nach Anspruch 1 oder 2, dadurch gekennzeichnet, daß als Alkalijodid Natrium- oder Kaliumjodid in Mengen von 0,01 bis 1, vorzugsweise 0,05 bis 1 und insbesondere 0,05 bis 0,5 Gew.-% bezogen auf die Epoxidkomponente, eingesetzt wird.

4. Verfahren nach Anspruch 1 bis 3, dadurch gekennzeichnet, daß als Polyole Ba) oder Ether Bb) Di, Tri- oder Polyglykole oder Mono- und/oder Diether eingesetzt werden.

5. Verfahren nach Anspruch 4, dadurch gekennzeichnet, daß Diethylen-, Triethylen-, Tetraethylen- und Dipropylenglykol, Ethylenglykolmonomethyl- und -monobutylether, Diethylenglykolmonomethyl- und -monobutylether, Diethylenglykoldimethyl- und diethylether, Triethylenglykolmono- und -dimethylether, Dimethoxyethan, Propylenglykolmonobutylether sowie Ethylen-Propylenglykol-monobutylether eingesetzt werden.

6. Verfahren nach einem oder mehreren der Ansprüche 1 bis 5, dadurch gekennzeichnet, daß die Verbindungen Ba) und Bb) in Mengen von 1 bis 95, vorzugsweise 1 bis 50 und insebesondere 10 bis 30 Gew.-%, bezogen auf die Epoxidkomponente, eingesetzt wird.

7. Verfahren nach einem oder mehreren der Ansprüche 1 bis 6, dadurch gekennzeichnet, daß die Umsetzung der Epoxidverbindung mit Kohlendioxid nur teilweise vorgenommen wird.

8. Verfahren nach einem oder mehreren der Ansprüche 1 bis 7, dadurch gekennzeichnet, daß Epoxidverbindungen umgesetzt werden, die mindestens eine endständige Epoxygruppe aufweisen und die aliphatische Epoxide mit mindestens 6 C-Atomen, Glycidol oder Epihalogenhydrine der Formel (2)

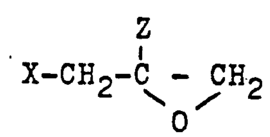

(2),

sind, oder die im Durchschnitt mindestens eine substituierte oder nicht substituierte Glycidylethergruppe der Formel (3)

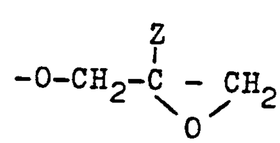

(3),

oder eine substituierte oder nicht substituierte Glycidylestergruppe der Formel (4)

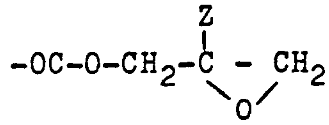 (4),

enthalten, wobei in den Formeln (2) bis (4) Z ein Wasserstoffatom, eine Methyl- oder Ethylgruppe und X ein Halogenatom oder eine OH-Gruppe ist, ferner epoxidierte, mehrfach ungesättigte Verbindungen und amid- oder urethangruppenhaltige Epoxide.

9. Verfahren nach Anspruch 8, dadurch gekennzeichnet, daß Poly(glycidylether, plastifizierte Epoxidharze mit endständigen Epoxidgruppen oder Glycidylester von gesättigten oder ethylenisch ungesättigten (Poly)Carbonsäuren umgesetzt werden.

10. Verwendung der nach dem Verfahren gemäß einem oder mehreren der Ansprüche 1 bis 9 erhaltenen 2-Oxo-1,3-dioxolane zur Herstellung von Urethangruppen enthaltenden Kunstharzen in Form von Überzügen oder Formkörpern.

**Revendications**

1. Procédé pour préparer des oxo-2 dioxolannes-1,3 par réaction d'époxydes avec du dioxyde de carbone en présente d'iodures de métaux alcalins, procédé caractérisé en ce qu'on fait réagir au moins un composé époxydique, en présence ou non d'un solvant inerte, soit avec une une association constituée A) d'au moins un iodure de metal alcalin et B) d'au moins un composé pris dans l'ensemble constitué par a) un polyol et b) un éther ou un polyéther répondant à l'une des formules suivantes:

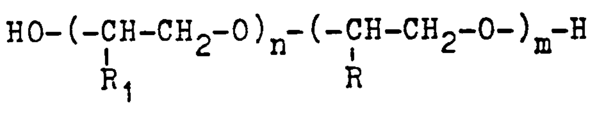 $n > 1$, $m \geqslant 0$ $B_a)$

und

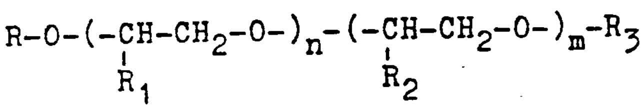 $n \geqslant 1$, $m \geqslant 0$ $B_b)$

dans lesquelles

les R représentent chacun, indépendamment les uns des autres, un alkyle en $C_1$—$C_{10}$, un aralkyle dont la partie alkyle contient de 1 à 10 atomes de carbone, ou un aryle,

$R_1$ et $R_2$ représentant chacun, indépendamment l'un de l'autre, l'hydrogène ou un alkyle en $C_1$—$C_{10}$ et $R_3$ a la signification indiquée pour R mais peut aussi représenter l'hydrogène,

soit, lorsque le composé époxydique présente déjà les caractéristiques structurales des composés Ba) ou Bb), avec l'iodure de métal alcalin A) seul, à des températures de 40 à 180°C, tout en faisant passer un courant de dioxyde de carbone sous la pression normale ou sous une pression légèrement supérieure à la pression normale, de manière à obtenir les carbonates organiques correspondants.

2. Procédé selon la revendication 1, caractérisé en ce qu'on opère sous une pression de 1 à 10 bar, de préférence de 1 à 5 bar ou, mieux encore, de 1 à 3 bar.

3. Procédé selon l'une des revendications 1 et 2, caractérisé en ce qu'on utilise, comme iodure de métal alcalin, l'iodure de sodium ou l'iodure de potassium en des quantités de 0,01 à 1% en poids, de préférence de 0,05 à 1 ou, mieux encore, de 0,05 à 0,5% en poids, par rapport à la composante époxydique.

4. Procédé selon l'une quelconque des revendications 1 à 3, caractérisé en ce qu'on utilise, comme polyols Ba) ou éthers Bb), des di-, tri- ou polyglycols ou les monoéthers et/ou diéthers.

5. Procédé selon la revendication 4, caractérisé en ce qu'on utilise le diéthylène-glycol, le triéthylèneglycol, le tétraéthylène-glycol, le dipropylène-glycol, l'éther monométhylique ou monobutylique de l'éthylèneglycol, le diméthoxy-éthane, l'éther monométhylique ou monobutylique du diéthylène-glycol, l'éther diméthylique ou diéthylique du diéthylène-glycol, l'éther monométhylique ou diméthylique du triéthylène-glycol, l'éther monobutylique du propylène-glycol ou des éthers monobutyliques d'éthylène-propylène-glycols.

6. Procédé selon l'une quelconque des revendications 1 à 5, caractérisé en ce que les composés Ba) et Bb) sont mis en jeu en des quantités de 1 à 95% en poids, de préférence de 1 à 50% ou, mieux encore, de 10 à 30% en poids, par rapport à la composante époxydique.

7. Procédé selon l'une quelconque des revendications 1 à 6, caractérisé en ce que la réaction du composé époxydique avec le dioxyde de carbone n'est effectuée que partiellement.

8. Procédé selon l'une quelconque des revendications 1 à 7, caractérisé en ce qu'on fair réagir des composés époxydiques qui contiennent au moins un radical époxy terminal et qui sont des époxydes aliphatiques renfermant au moins 6 atomes de carbone, le glycidol ou des épihalogénhydrines répondant à la formule (2)

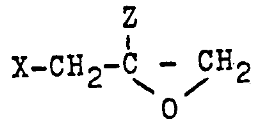

(2),

ou qui contiennent en moyenne au moins un radical d'éther glycidylique, substitué ou non, répondant à la formula (3)

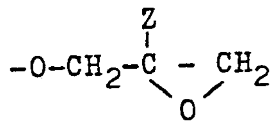

(3),

ou an moins un radical d'ester glycidylique, substitué ou non, répondant à la formule (4)

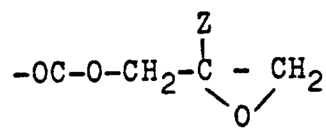

(4),

formules dans lesquelles Z représente un atome d'hydrogène ou un radical méthyle ou éthyle, et X un atome d'halogène ou un radical -OH, également des composés multi-insaturés époxydés et des époxydes contenant des radicaux d'amides ou d'uréthannes.

9. Procédé selon la revendication 8, caractérisé en ce qu'on fait réagir des éthers (poly)glycidyliques, des résines époxydiques plastifiées à radicaux époxy terminaux, ou des esters glycidyliques dérivant d'acides (poly)carboxyliques saturés ou éthyléniques.

10. Application des oxo-2 dioxolannes-1,3 obtenus par le procédé spécifié à l'une quelconque des revendications 1 à 9, à la préparation de résines synthétiques contenant des radicaux d'uréthannes, sous la forme de revêtements ou d'objets moulés.

**Claims**

1. A process for the preparation of 2-oxo-1,3-dioxolanes by a reaction of epoxides with carbon dioxide in the presence of alkali iodides, which comprises reacting at least one epoxy compound in the presence or absence of an inert solvent either with a combination of A) at least one alkali iodide and B) at least one compound of the group a) polyol and b) an ether or polyether having the structures

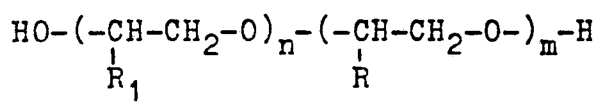

$n > 1$

$m \geqslant 0$ $B_a)$

und

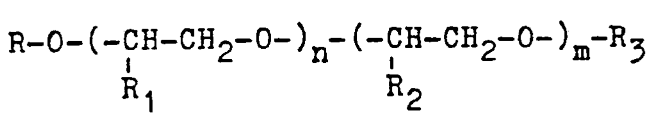

$n \geqslant 1$

$m \geqslant 0$ $B_b)$

in which

R is identical or different and is alkyl, aralkyl containing 1—10 C atoms in the alkyl group in each case, or aryl,

$R_1$, $R_2$ are identical or different and are hydrogen or alkyl containing 1—10 C atoms and

$R_3$ is identical to R, but may also be hydrogen, or, if the epoxy compound already has the structural features of the compounds Ba) or Bb), with alkali iodide A) alone, at temperatures from 40 to 180°C while introducing carbon dioxide at normal pressure or at slightly increased pressure, for form the corresponding organic carbonates.

2. The process as claimed in claim 1, wherein a pressure of 1 to 10, preferably 1 to 5, in particular 1 to 3 bar is employed.

3. The process as claimed in claim 1 or 2, wherein sodium or potassium iodide in quantities of 0.01 to 1, preferably 0.05 to 1 and in particular 0.05 to 0.5% by weight, referred to the epoxy component, is used as alkali iodide.

4. The process as claimed in claim 1 or 3, wherein di-, tri- or polyglycols or mono- and/or diethers are used as polyols Ba) or ethers Bb).

5. The process as claimed in claim 4, wherein diethylene, triethylene, tetraethylene and dipropylene

glycol, ethylene glycol monomethyl and monobutyl ether, dimethoxyethane, diethylene glycol monoethyl and monobutylether, diethylene glycol dimethyl and diethyl ether, triethylene glycol monomethyl and dimethyl ether, propylene glycol monobutylether and also ethylene propylene glycol monobutylethyl are used.

6. The process as claimed in one or more of the claims 1 to 5, wherein the compounds Ba) and Bb) are used in quantities of 1 to 95, preferably 1 to 50 and in particular 10 to 30% by weight, referred to the epoxy component.

7. The process as claimed in one or more of the claims 1 to 6, wherein the reaction of the epoxy compound is only partly carried out with carbon dioxide.

8. The process as claimed in one or more of the claims 1 to 7, wherein epoxy compounds are reacted which have at least one terminal epoxy group and are aliphatic epoxides having at least 6 C atoms, glycidol or epihalohydrines of the formula (2)

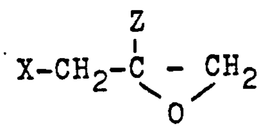

(2),

or contain an average at least one substituted or unsubstituted glycidyl ether group of the formula (3)

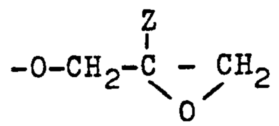

(3),

or a substituted or unsubstituted glycidyl ester group of the formula (4).

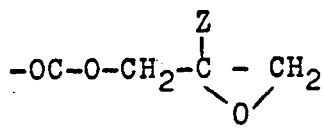

(4),

Z is the formulae (3) to (4) being a hydrogen atom, a methyl or ethyl group and X being a halogen atom or an OH group, furthermore epoxidized, multiply unsaturated compounds and epoxides containing amide or urethane groups.

9. The process as claimed in claim 8, wherein (poly)glycidyl ethers, plasticized epoxy resins with terminal epoxy groups or glycidyl esters of saturated or ethylenically unsaturated (poly)carboxylic acids are reacted.

10. The use of the 2-oxo-1,3-dioxolanes obtained by the process as claimed in one or more of the claims 1 to 9 to prepare synthetic resins, containing urethane groups, in the form of coatings or molded bodies.